# EUROPEAN PATENT APPLICATION

(11) **EP 1 275 714 A1**
(43) Date of publication of application: **15.01.2003**
(21) Application number: 01919886.0
(22) Date of filing: 11.04.2001
(51) Int. Cl.: C12N 15/09, C12N 9/10, C12P 19/18

(54) **ALPHA 1,2-FUCOSYLTRANSFERASE AND PROCESS FOR PRODUCING FUCOSE-CONTAINING COMPLEX CARBOHYDRATE**

(30) Priority: 11.04.2000 JP 2000109148
(71) Applicant: KYOWA HAKKO KOGYO CO., LTD., Chiyoda-ku, Tokyo 100-8185 (JP)
(72) Inventor: ENDO, Tetsuo, Tokyo Research Laboratories, Machida-shi, Tokyo 194-8533 (JP); KOIZUMI, Satoshi, Tokyo Research Laboratories, Machida-shi, Tokyo 194-8533 (JP)
(74) Representative: VOSSIUS & PARTNER
(86) International application number: JP0103109
(87) International publication number: WO01077313

(57) **Abstract**

According to the present invention, α1,2-fucosyltransferase can be expressed in a large amount by using *Escherichia coli*. Also, a fucose-containing complex carbohydrate can be economically produced in a large amount by contacting the *Escherichia coli* expressing the α1,2-fucosyltransferase and a microorganism capable of producing GDP-fucose from a sugar and GTP, a microorganism capable of producing GTP from a precursor and an acceptor complex carbohydrate in an aqueous medium.

SEQUENCE LISTING

<110> KYOWA HAKKO KOGYO CO.,LTD

<120> Production of alpha 1,2-fucosyltransferase and complex carbohydrate
containing fucose

<130> 11296W01

<140>
<141>

<150> JP 00/109148
<151> 2000-4-11

<160> 12

<170> PatentIn Ver. 2.0

<210> 1
<211> 861
<212> DNA
<213> Bacteroides fragilis

<220>
<221> CDS
<222> (1)..(861)

<400> 1
atg tta tat gta att tta cgt gga cga tta ggt aat aat ctt ttt cag 48
Met Leu Tyr Val Ile Leu Arg Gly Arg Leu Gly Asn Asn Leu Phe Gln
1               5                   10                  15

ata gca act gcc gct tcg ttg act cag aat ttt ata ttt tgt aca gta 96
Ile Ala Thr Ala Ala Ser Leu Thr Gln Asn Phe Ile Phe Cys Thr Val
            20                  25                  30

aat aag gac caa gag aga cag gtc ctt ttg tat aag gat tct ttt ttt 144
Asn Lys Asp Gln Glu Arg Gln Val Leu Leu Tyr Lys Asp Ser Phe Phe
        35                  40                  45

aaa aat ata aaa gtt atg aag ggg gtt cct gat ggc ata cca tat tac 192
Lys Asn Ile lys Val Met Lys Gly Val Pro Asp Gly Ile Pro Tyr Tyr
    50                  55                  60

aaa gaa cca ttc cat gaa ttt agc aga att cct tat gaa gaa gga aag 240
Lys Glu Pro Phe His Glu Phe Ser Arg Ile Pro Tyr Glu Glu Gly Lys
65                  70                  75                  80

gat ctc att att gat gga tat ttc caa tca gaa aag tac ttt aaa aga 288
Asp Leu Ile Ile Asp Gly Tyr Phe Gln Ser Glu Lys Tyr Phe Lys Arg
                85                  90                  95

agt gtc gta tta gat ctt tat aga ata act gat gag cta agg aag aaa 336
Ser Val Val Leu Asp Leu Tyr Arg Ile Thr Asp Glu Leu Arg Lys Lys
            100                 105                 110

ata tgg aat att tgt gga aat att tta gaa aag gga gaa act gtg agt 384
Ile Trp Asn Ile Cys Gly Asn Ile Leu Glu Lys Gly Glu Thr Val Ser
        115                 120                 125

att cat gtt aga aga ggt gat tac ttg aag ctg cca cat gca tta cca 432
Ile His Val Arg Arg Gly Asp Tyr Leu Lys Leu Pro His Ala Leu Pro
    130                 135                 140

ttt tgt gga aag tca tac tat aag aat gct att caa tat att ggt gag 480
Phe Cys Gly Lys Ser Tyr Tyr Lys Asn Ala Ile Gln Tyr Ile Gly Glu
145                 150                 155                 160

gat aaa ata ttc att att tgt agt gat gat atc gat tgg tgt aaa aaa 528
Asp Lys Ile Phe Ile Ile Cys Ser Asp Asp Ile Asp Trp Cys Lys Lys
                165                 170                 175

aac ttt ata gga aaa aga tat tac ttc ata gag aac act act cct tta 576
Asn Phe Ile Gly Lys Arg Tyr Tyr Phe Ile Glu Asn Thr Thr Pro Leu
            180                 185                 190

cta gat tta tat atc caa tcc ttg tgc act cac aat att ata agt aat 624
Leu Asp Leu Tyr Ile Gln Ser Leu Cys Thr His Asn Ile Ile Ser Asn
        195                 200                 205

agc tct ttt agt tgg tgg gga gca tgg ctt aat gaa aat agt aat aaa     672
Ser Ser Phe Ser Trp Trp Gly Ala Trp Leu Asn Glu Asn Ser Asn Lys
    210                 215                 220

att gtt att gca cct caa atg tgg ttt ggc att tct gtg aag ttg ggt     720
Ile Val Ile Ala Pro Gln Met Trp Phe Gly Ile Ser Val Lys Leu Gly
225                 230                 235                 240

gtt agt gat tta ttg cct gtc agt tgg gtt cga ctt cct aat aat tat     768
Val Ser Asp Leu Leu Pro Val Ser Trp Val Arg Leu Pro Asn Asn Tyr
                245                 250                 255

act tta gga aga tat tgt ttt gct cta tat aaa gta gtt gag gac tat     816
Thr Leu Gly Arg Tyr Cys Phe Ala Leu Tyr Lys Val Val Glu Asp Tyr
            260                 265                 270

tta tta aat att ctg cga tta ata tgg aaa aga aag aag aat atg         861
Leu Leu Asn Ile Leu Arg Leu Ile Trp Lys Arg Lys Lys Asn Met
        275                 280                 285

<210> 2
<211> 287
<212> PRT
<213> Bacteroides fragilis

<400> 2
Met Leu Tyr Val Ile Leu Arg Gly Arg Leu Gly Asn Asn Leu Phe Gln
1               5                   10                  15

Ile Ala Thr Ala Ala Ser Leu Thr Gln Asn Phe Ile Phe Cys Thr Val
            20                  25                  30

Asn Lys Asp Gln Glu Arg Gln Val Leu Leu Tyr Lys Asp Ser Phe Phe
        35                  40                  45

Lys Asn Ile lys Val Met Lys Gly Val Pro Asp Gly Ile Pro Tyr Tyr
    50                  55                  60

Lys Glu Pro Phe His Glu Phe Ser Arg Ile Pro Tyr Glu Glu Gly Lys
65                  70                  75                  80

Asp Leu Ile Ile Asp Gly Tyr Phe Gln Ser Glu Lys Tyr Phe Lys Arg
                85                  90                  95

Ser Val Val Leu Asp Leu Tyr Arg Ile Thr Asp Glu Leu Arg Lys Lys
            100                 105                 110

Ile Trp Asn Ile Cys Gly Asn Ile Leu Glu Lys Gly Glu Thr Val Ser
        115                 120                 125

Ile His Val Arg Arg Gly Asp Tyr Leu Lys Leu Pro His Ala Leu Pro
    130                 135                 140

Phe Cys Gly Lys Ser Tyr Tyr Lys Asn Ala Ile Gln Tyr Ile Gly Glu
145                 150                 155                 160

Asp Lys Ile Phe Ile Ile Cys Ser Asp Asp Ile Asp Trp Cys Lys Lys
                165                 170                 175

Asn Phe Ile Gly Lys Arg Tyr Tyr Phe Ile Glu Asn Thr Thr Pro Leu
            180                 185                 190

Leu Asp Leu Tyr Ile Gln Ser Leu Cys Thr His Asn Ile Ile Ser Asn
        195                 200                 205

Ser Ser Phe Ser Trp Trp Gly Ala Trp Leu Asn Glu Asn Ser Asn Lys
    210                 215                 220

Ile Val Ile Ala Pro Gln Met Trp Phe Gly Ile Ser Val Lys Leu Gly
225                 230                 235                 240

Val Ser Asp Leu Leu Pro Val Ser Trp Val Arg Leu Pro Asn Asn Tyr
                245                 250                 255

Thr Leu Gly Arg Tyr Cys Phe Ala Leu Tyr Lys Val Val Glu Asp Tyr
            260                 265                 270

Leu Leu Asn Ile Leu Arg Leu Ile Trp Lys Arg Lys Lys Asn Met
        275                 280                 285

<210> 3
<211> 35
<212> DNA
<213> Artificial Sequence

<220>
<223> Description of Artificial Sequence: Synthetic DNA

<400> 3
taataagctt atgttatatg taattttacgt ggacg                                35

<210> 4
<211> 37
<212> DNA
<213> Artificial Sequence

<220>
<223> Description of Artificial Sequence: Synthetic DNA

<400> 4
agtcaggatc cttttcacat attcttcttt cttttcc                               37

<210> 5
<211> 34
<212> DNA
<213> Artificial Sequence

<220>
<223> Description of Artificial Sequence: Synthetic DNA

<400> 5
ccgcaagatc tcgtaaaaaa gggtatcgat aagc                                  34

<210> 6
<211> 20
<212> DNA
<213> Artificial Sequence

<220>
<223> Description of Artificial Sequence: Synthetic DNA

<400> 6
gagctgactg ggttgaaggc                                                    20


<210> 7
<211> 27
<212> DNA
<213> Artificial Sequence

<220>
<223> Description of Artificial Sequence: Synthetic DNA

<400> 7
gaatctagaa tgtcaaaagt cgctctc                                            27


<210> 8
<211> 25
<212> DNA
<213> Artificial Sequence

<220>
<223> Description of Artificial Sequence: Synthetic DNA

<400> 8
ctcaagctta tgactccagc gcgat                                              25


<210> 9
<211> 24
<212> DNA
<213> Artificial Sequence

<220>
<223> Description of Artificial Sequence: Synthetic DNA

<400> 9
caagaattct catgtttgac agct                                               24

<210> 10
<211> 48
<212> DNA
<213> Artificial Sequence

<220>
<223> Description of Artificial Sequence: Synthetic DNA

<400> 10
cattctagac ctccttaatt cgcgaaaatg gatcgatacc ctttttac                48

<210> 11
<211> 27
<212> DNA
<213> Artificial Sequence

<220>
<223> Description of Artificial Sequence: Synthetic DNA

<400> 11
gtcatcgata tgagtaaaca acgagtt                                       27

<210> 12
<211> 25
<212> DNA
<213> Artificial Sequence

<220>
<223> Description of Artificial Sequence: Synthetic DNA

<400> 12
ataaactcga gagagacaag cggag                                         25

## Description

### TECHNICAL FIELD

The present invention relates to a process for producing α1,2-fucosyltransferase and a process for producing a fucose-containing complex carbohydrate, using a transformant which comprises a recombinant DNA comprising a DNA encoding a protein having α1,2-fucosyltransferase activity.

### BACKGROUND OF THE INVENTION

α1,2-Fucosyltransferase genes derived from animals have so far been obtained [*Proc. Natl. Acad. Sci., USA*, 87, 6674 (1990), *Immunogenetics,* 44, 76 (1996), *J. Biol. Chem.,* 264, 3436 (1989), *J. Biol. Chem.,* 264, 11158 (1989), J. *Biol. Chem.,* 267, 2737 (1992), *J. Biol. Chem.,* 270, 8844 (1995), *J. Biol. Chem*., 270, 4640 (1995), *J. Biochem*., 118, 541 (1995), *J. Biol. Chem*., 271, 16975 (1996)] and however, there are no reports showing that the enzyme was expressed as a protein having the activity using a microorganism such as *Escherichia coli* or the like.

On the other hand, in microorganisms, an α1,2-fucosyltransferase gene has been obtained from *Helicobacter pylori* and there is a report showing that α1,2-fucosyltransferase was expressed in *Escherichia coli* using the gene, but the enzyme activity is extremely weak even under control of the gene by a strong promoter [*Microbiology*, 145, 3245 (1999)]. Also, there is a report showing that a product of the wcfB gene among the extracellular polysaccharide biosynthesis genes in *Bacteroides fragilis* has homology with known α1,2-fucosyltransferase [*Infect. Immun.,* 67, 3525(1999)], but there is no case of detecting the activity of the gene product.

Fucose-containing complex carbohydrates have been known as blood group antigen sugar chains, and it has been recently clarified that those sugar chains undergo structural changes with canceration of cells *[Analytical Biochemistry,* 251, 89 (1997)], so that the applications as a tumor marker and medicaments are expected. Also, oligosaccharides are contained abundantly in human milk, and fucose-containing complex carbohydrates (fucosyllactose is one of the main components) occupy at least 70% of the total oligosaccharides [*Glycoblology*, 8, 615 (1998)]. Since it is known that a complex carbohydrate having a Fucα1-2Gal structure which is also contained in the milk oligosaccharides inhibits infection of *Candida albicans [Infect. Immun*., 59, 1650 (1991)], fucose-containing complex carbohydrates are considered to be strong candidates for safe infection preventing agents.

However, as the production of fucose-containing complex carbohydrates such as fucosyllactose and the like, an extraction method from human milk [*J. Chromatography,* 211, 170 (1981)], a production process which uses transgenic animals *[J. Biol. Chem*., 270, 29515 (1995), U.S.P. 5,700,671] and a method using an enzyme (U.S.P. 5,583,042) have been reported, but all of them have problems in terms of cost and productivity so that an industrial production process has not been established yet.

### DISCLOSURE OF THE INVENTION

An object of the present invention is to provide a process for producing α1,2-fucosyltransferase and an industrial process for producing a fucose-containing complex carbohydrate.

In order to solve the above problems, the present inventors have conducted intensive studies and succeeded in plentifully expressing α1,2-fucosyltransferase gene derived from a microorganism belonging to the genus *Bacteroides* in *Escherichia coli,* whose activity has not so far been expressed in microorganisms such as *Escherichia coli* and the like. The present inventors have been further found that the enzyme uses lacto-N-neotetraose, lactose and the like as the substrate and that a fucose-containing complex carbohydrate can be produced in a large amount using the enzyme.

Accordingly, the present invention relates to the following items (1) to (23).
(1) A process for producing a fucose-containing complex carbohydrate, which comprises selecting, as an enzyme source, a culture of a transformant expressing a protein having α1,2-fucosyltransferase activity derived from a microorganism belonging to the genus *Bacteroides* or a treated product of the culture; allowing the enzyme source, an acceptor complex carbohydrate and guanosine diphosphofucose (hereinafter referred to as "GDP-fucose") to be present in an aqueous medium; transferring fucose to the acceptor complex carbohydrate by α1,2 linkage to form and accumulate the fucose-containing complex carbohydrate in the aqueous medium; and recovering the fucose-containing complex carbohydrate from the aqueous medium.
(2) A process for producing the fucose-containing complex carbohydrate according to (1), which comprises selecting, as enzyme sources, a culture of a microorganism capable of producing guanosine 5'-triphosphate (hereinafter referred to as "GTP") from a precursor or a treated product of the culture, a culture of a microorganism capable of producing GDP-fucose from a sugar and GTP or a treated product of the culture and a culture of a transformant expressing a protein having α1,2-fucosyltransferase activity derived from a microorganism belonging to the genus Bacteroides or a treated product of the culture; allowing the enzyme sources, the precursor, the sugar and an acceptor complex carbohydrate to be present in an aqueous medium; allowing the fucose-containing complex carbohydrate to form and accumulate in the aqueous medium; and recovering the fucose-containing complex carbohydrate from the aqueous medium.
(3) A process for producing a protein having α1,2-fucosyltransferase activity, which comprises culturing a transformant expressing a protein having α1,2-fucosyltransferase activity derived from a microorganism belonging to the genus *Bacteroides* in a medium; allowing the protein having α1,2-fucosyltransferase activity to form and accumulate in the culture; and recovering the protein from the culture.
(4) The process according to any one of (1) to (3), wherein the transformant is a transformant obtained by introducing a recombinant DNA into a microorganism.
(5) The process according to (4), wherein the microorganism is a microorganism belonging to the genus *Escherichia*.
(6) The process according to (5), wherein the microorganism belonging to the genus *Escherichia* is *Escherichia coli*.
(7) The process according to (4), wherein the recombinant DNA comprises a DNA encoding a protein having α1,2-fucosyltransferase activity derived from a microorganism belonging to the genus *Bacteroides*.
(8) The process according to (7), wherein the DNA encoding a protein having α1,2-fucosyltransferase activity derived from a microorganism belonging to the genus *Bacteroides* is a DNA described in the following [1] or [2]:
   [1] a DNA comprising the nucleotide sequence represented by SEQ ID NO:1;
   [2] a DNA which comprises a nucleotide sequence in which at least one nucleotide in the DNA consisting of the nucleotide sequence represented by SEQ ID NO:1 is deleted, substituted or added, and encodes a protein having α1,2-fucosyltransferase activity.
(9) The process for producing a fucose-containing complex carbohydrate according to (1) or (2), wherein the acceptor complex carbohydrate is a complex carbohydrate containing an oligosaccharide consisting of 10 or less sugar having galactose in the non-reducing terminal.
(10) The process for producing a fucose-containing complex carbohydrate according to (9), wherein the oligosaccharide has a lactose, N-acetyllactosamine, lacto-N-neotetraose, lacto-N-tetraose, Le^{x} or Le^{a} structure in the non-reducing terminal.
(11) The process for producing a fucose-containing complex carbohydrate according to (1) or (2), wherein the treated product of the culture is a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells or an enzyme preparation obtained by extracting the cells.
(12) The process for producing a fucose-containing complex carbohydrate according to (2), wherein the precursor is guanine, xanthine, hypoxanthine, guanosine, xanthosine, inosine, guanosine-5'-monophosphate, xanthosine-5'-monophosphate or inosine-5'-monophosphate.
(13) The process for producing a fucose-containing complex carbohydrate according to (2), wherein the sugar is selected from glucose, fructose and mannose.
(14) The process for producing a fucose-containing complex carbohydrate according to (2), wherein the microorganism capable of producing GTP from a precursor is a microorganism selected from microorganisms belonging to the genus *Corynebacterium.*
(15) The process for producing a fucose-containing complex carbohydrate according to (14), wherein the microorganism belonging to the genus *Corynebacterium* is *Corynebacterium ammoniagenes.*
(16) The process for producing a fucose-containing complex carbohydrate according to (2), wherein the microorganism capable of producing GDP-fucose from a sugar and GTP consists of at least one microorganism.
(17) The process for producing a fucose-containing complex carbohydrate according to (16), wherein the microorganism is at least one microorganism selected from microorganisms belonging to the genus *Escherichia* and genus *Corynebacterium*.
(18) The process for producing a fucose-containing complex carbohydrate according to (17), wherein the microorganism belonging to the genus *Escherichia* is *Escherichia coli.*
(19) The process for producing a fucose-containing complex carbohydrate according to (17), wherein the microorganism belonging to the genus *Corynebacterium* is *Corynebacterium ammoniagenes*.
(20) The process for producing a fucose-containing complex carbohydrate according to (2), wherein the microorganism capable of producing GDP-fucose from a sugar and GTP is a microorganism having potent activity of at least one enzyme selected from the group consisting of glucokinase, phosphomannomutase, mannose-1-phosphate guanylyltransferase, phosphoglucomutase, phosphofructokinase, GDP-mannose 4,6-dehydratase and GKDM epimerase/reductase.
(21) The process for producing a fucose-containing complex carbohydrate according to (20), wherein the microorganism is at least one microorganism containing a recombinant DNA which comprises a DNA fragment comprising a gene selected from the group consisting of a gene encoding glucokinase (hereinafter referred to as "glk gene"), a gene encoding phosphomannomutase (hereinafter referred to as "manB gene"), a gene encoding mannose-1-phosphate guanylyltransferase (hereinafter referred to as "manC gene"), a gene encoding phosphoglucomutase (hereinafter referred to as "pgm gene"), a gene encoding phosphofructokinase (hereinafter referred to as "pfk gene"), a gene encoding. GDP-mannose 4,6-dehydratase (hereinafter referred to as "gmd gene") and a gene encoding GKDM epimerase/reductase (hereinafter referred to as "wcaG gene"); and a vector.
(22) The process for producing a fucose-containing complex carbohydrate according to (21), wherein the glk gene, the manB gene, the manC gene, the pgm gene, the pfk gene, the gmd gene and the wcaG gene are genes derived from *Escherichia coli.*
(23) An agent for synthesizing a fucose-containing complex carbohydrate, which comprises a protein selected from the following [1] to [3] as an active ingredient:
   [1] a protein having α1,2-fucosyltransferase activity derived from a microorganism belonging to the genus *Bacteroides;*
   [2] a protein comprising the amino acid sequence represented by SEQ ID NO:2;
   [3] a protein which comprises an amino acid sequence in which at least one amino acid of the protein consisting of the amino acid sequence represented by SEQ ID NO:2 is deleted, substituted or added, and has α1,2-fucosyltransferase activity.

The present invention is described below in detail.

The DNA used in the production processes of the present invention encoding a protein having α1,2-fucosyltransferase activity is a DNA encoding a protein having α1,2-fucosyltransferase activity derived from a microorganism belonging to the genus *Bacteroides*. Example includes a DNA encoding a protein having α1,2-fucosyltransferase activity derived from *Bacteroides fragilis.* Also, the DNA used in the production processes of the present invention encoding a protein having α1,2-fucosyltransferase activity includes
(1) a DNA which comprises a nucleotide sequence in which at least one nucleotide of the nucleotide sequence of the DNA encoding a protein having α1,2-fucosyltransferase activity derived from a microorganism belonging to the genus *Bacteroides* is deleted, substituted or added, and has α1,2-fucosyltransferase activity;
(2) among the DNA in which at least one nucleotide is substituted in the above (1), a DNA comprising a nucleotide sequence obtained by carrying out nucleotide substitution of at lease one codon with other codon(s) having high frequency of codon usage in a host organism for expressing the DNA;
and the like.

The DNA of the above (1) in which at least one nucleotide is deleted, substituted or added can be obtained by using a site-directed mutagenesis described in *Molecular Cloning, A Laboratory Manual,* Second Edition, (1989) (hereinafter referred to as *"Molecular Cloning,* Second Edition"), *Current Protocols in Molecular Biology,* John Wiley & Sons (1987-1997) (hereinafter referred to as *"Current Protocols in Molecular Biology*") or the like. The number of deleted, substituted or added amino acids generated in the protein encoded by the mutation-introduced DNA is not particularly limited but, for example, is from 1 to 20, preferably from 1 to 15, and more preferably from 1 to 5.

Also, in order to allow the DNA to encode a protein having α1,2-fucosyltransferase activity, it is preferable that the mutation-introduced DNA has at least 60% or more, generally 80% or more, particularly 95% or more, of identity with the amino acid sequence represented by SEQ ID NO:2, when calculated using BLAST [*J. Mol. Biol*., 215, 403 (1990)], FASTA *[Methods in Enzymology*, 183, 63 (1990)] or the like.

Also, the host organism of the transformant used in the production process of the present invention is not particularly limited, so long as it can express a DNA which encodes a protein having α1,2-fucosyltransferase activity and is comprised in a recombinant DNA contained in the transformant, and individual of an organism, tissues of an organism, cultured cells of an organism, cells of an microorganism can be used as the host organism. The host organism is preferably a microorganism, more preferably *Escherichia coli.* The codon having high frequency of codon usage is a codon having a codon usage in the host cell of at least 10% or more, preferably 15% or more, more preferably 20% or more, corresponding to each amino acid.

The method for producing a codon-modified DNA includes a method in which a DNA is artificially synthesized by designing and synthesizing a primer DNA based on a nucleotide sequence of the desired DNA and carrying out PCR [*PCR Protocols,* Humana Press (1993)] using the synthetic DNAs in addition to the above site-directed mutagenesis.

Examples of the process for producing the protein of the present invention having α1,2-fucosyltransferase activity include a method in which a host organism is transformed using a recombinant DNA containing a DNA encoding a protein having α1,2-fucosyltransferase activity in accordance with the method described in *Molecular Cloning,* Second Edition, the thus obtained transformant is cultured in an appropriate medium in which the transformant can be grown to thereby accumulate the protein in the culture, and then the protein is recovered from the culture. In addition, the protein can be recovered from the culture, for example, by a method in which the protein is solubilized and then isolated and purified by an ion exchange, gel filtration, hydrophobic chromatography or the like, or by a combination of the chromatographic techniques.

Examples of the process for producing the fucose-containing complex carbohydrate of the present invention include a method in which it is produced by using a culture of a transformant capable of producing the protein having α1,2-fucosyltransferase activity, a treated product of the culture or purified product of the protein as the enzyme source, and allowing the enzyme source to coexist with an acceptor complex carbohydrate and GDP-fucose in an aqueous medium.

Also, examples of the process for producing the fucose-containing complex carbohydrate of the present invention include a method in which it is produced using GDP-fucose which is obtained by allowing a culture of a microorganism capable of producing GTP from a precursor or a treated product of the culture, and a culture of a microorganism(s) constituted by at least one microorganism capable of producing GDP-fucose from a sugar and GTP or a treated product of the culture, to be present in an aqueous medium. The precursor as used herein is not particularly limited, so long as it can be converted into GTP by a microorganism. Preferred examples of the precursor include guanine, xanthine, hypoxanthine, guanosine, xanthosine, inosine, guanosine-5'-monophosphate, xanthosine-5'-monophosphate, inosine-5'-monophosphate and the like. The sugar is not particularly limited, so long as it can be converted into GDP-fucose. Preferred examples of the sugar include glucose, fructose and mannose. The acceptor complex carbohydrate is not particularly limited, so long as it can become a substrate of the protein having α1,2-fucosyltransferase activity encoded by the recombinant DNA of the present invention. Preferred examples include a sugar having galactose in the non-reducing terminal, and more preferred examples include an oligosaccharide having a lactose, N-acetyllactosamine, lacto-N-neotetraose, lacto-N-tetraose, Le^{x} or Le^{a} structure in the non-reducing terminal.

The microorganism capable of producing GTP from a precursor is not limited, so long as it is a microorganism having such ability, but is preferably a microorganism belonging to the genus *Corynebacterium,* more preferably *Corynebacterium ammoniagenes*. The microorganism capable of producing GDP-fucose from a sugar and GTP is a microorganism having potent activity of at least one enzyme selected from the group consisting of glucokinase, phosphomannomutase, mannose-1-phosphate guanylyltransferase, phosphoglucomutase, phosphofructokinase, GDP-mannose 4,6-dehydratase and GKDM epimerase/reductase, preferably a microorganism(s) consisting of at least one microorganism which contains a recombinant DNA which comprises a DNA fragment comprising at least one gene selected from the group consisting of genes encoding glucokinase, phosphomannomutase, mannose-1-phosphate, phosphoglucomutase, phosphofructokinase, GDP-mannose 4,6-dehydratase and GKDM epimerase/reductase and a vector, and more preferably at least one recombinant *Escherichia coli* comprising the glk gene, the manB gene, the manC gene, the pgm gene, the pfk gene, the gmd gene and the wcaG gene derived from *Escherichia coli.*

The thus produced fucose-containing complex carbohydrate can be recovered by general chromatography using activated charcoal, an ion exchange resin or the like.

The present invention is explained below in detail.

### [1] Production of a transformant expressing a protein having α1,2-fucosyltransferase activity of the present invention

### (1) Preparation of DNA encoding a protein having α1,2-fucosyltransferase activity

The DNA encoding a protein having α1,2-fucosyltransferase activity can be prepared using microorganism belonging to the genus *Bacteroides.* Examples of the microorganism belonging to the genus *Bacteroides* include *Bacteroides fragilis* (ATCC 25285) and the like. The microorganism belonging to the genus *Bacteroides* is cultured by a known method [e.g., *Infect. Immun*., 67, 3525 (1999)]. After culturing, chromosomal DNA of the microorganism is isolated and purified by a known method (*e.g.*, method described in *Current Protocols in Molecular Biology*).

A fragment containing the DNA encoding a protein having α1,2-fucosyltransferase activity can be obtained by PCR using primers prepared based on known nucleotide sequences *[Infect. Immun*., 67, 3525 (1999)] and the chromosomal DNA as the template.

Also, the desired DNA can be obtained by a hybridization method using a synthetic DNA designed by known sequences as a probe, or the like.

Furthermore, the DNA encoding a protein having α1,2-fucosyltransferase activity can be artificially synthesized by PCR or the like according to the usual method [e.g., *PCR Protocols,* Human Press (1993), etc.] using a synthetic DNA.

### (2) Cloning of DNA encoding a protein having α1,2-fucosyltransferase activity and confirmation of its nucleotide sequence

The DNA encoding a protein having α1,2-fucosyltransferase activity prepared in the above (1) is ligated with a vector according to the usual method as it is or after digestion with an appropriate restriction enzyme.

The vector with which the DNA is ligated may be any vector, such as a phage vector, a plasmid vector or the like, so long as it can replicate autonomously in *Escherichia coli* K12. Examples include ZAP Express [manufactured by Stratagene, *Strategies,* 5, 58 (1992)], pBluescript II SK(+) [manufactured by Stratagene, *Nucleic Acids Res.,* 17, 9494 (1989)], λzap II (manufactured by Stratagene), λgt10 and λgt11 *[DNA Cloning, A Practical Approach,* 1, 49 (1985)], λTriplEx (manufactured by Clontech), λExCell (manufactured by Pharmacia), pT7T318U (manufactured by Pharmacia), pcD2 [*Mol. Cell. Biol.,* 3, 280 (1983)], pUC18 [*Gene*, 33, 103 (1985)] and the like.

Any microorganism belonging to *Escherichia coli* can be used for the host of the recombinant DNA obtained by ligating the DNA encoding a protein having α1,2-fucosyltransferase activity prepared in the above (1) with the vector, so long as it is a microorganism belonging to *Escherichia coli*. Examples include *Escherichia coli* XL1-Blue MRF' [manufactured by Stratagene, *Strategies*, 5, 81 (1992)], *Escherichia coli* C600 *[Genetics,* 39, 440 (1954)], *Escherichia coli* Y1088 *[Science,* 222, 778 (1983)], *Escherichia coli* Y1090 *[Science,* 222, 778 (1983)], *Escherichia coli* NM522 [*J. Mol. Biol*., 166, 1 (1983)], *Escherichia coli* K802 [*J. Mol. Biol*., 16, 118 (1966)], *Escherichia coli* JM105 [*Gene*, 38, 275 (1985)] and the like.

Any method can be used in the method for introducing the recombinant DNA, so long as it is a method for introducing DNA into the above host cell. Examples include a method using a calcium ion [*Proc. Natl. Acad. Sci. USA*, 69, 2110 (1972)], a protoplast method (Japanese Published Unexamined Patent Application No. 248394/88), electroporation *[Nucleic Acid Res.,* 16, 6127 (1988)] and the like.

The nucleotide sequence of the DNA encoding α1,2-fucosyltransferase contained in the recombinant DNA can be determined by extracting the recombinant DNA from the thus obtained transformant. For the determination of the nucleotide sequence, a conventional method, such as the dideoxy method [*Proc. Natl. Acad. Scl. USA*, 74, 5463 (1977)] or an apparatus for nucleotide sequence analysis, such as DNA Sequencer 373A (manufactured by Perkin-Elmer) or the like, can be used.

Examples of transformant containing the thus obtained recombinant DNA include *Escherichia coli* NM522/pMG3 containing a plasmid DNA obtained by ligating a DNA comprising the nucleotide sequence represented by SEQ ID NO:1 with a vector DNA.

### [2] Production of a protein having α1,2-fucosyltransferase activity

The protein having α1,2-fucosyltransferase activity can be produced by expressing the DNA obtained above [1] in a host cell, for example, as shown below, using a method described in *Molecular Cloning,* Second Edition; *Current Protocols in Molecular Biology* or the like.

Specifically, based on the DNA encoding a protein having α1,2-fucosyltransferase activity obtained above [1], a DNA fragment of an appropriate length containing a portion which encodes the protein can be prepared, if necessary, and a recombinant DNA is constructed by inserting the DNA fragment into the downstream of the promoter in an appropriate expression vector. A protein having α1,2-fucosyltransferase activity can be produced by introducing the recombinant DNA into a host cell suitable for the expression vector to obtain a transformant, and culturing the transformant in a medium to accumulate the protein in the culture.

Any bacteria, yeasts, animal cells, insect cells, plant cells, and the like can be used as the host cell, so long as it can express the desired gene.

Examples of the expression vector include those which can replicate autonomously in the above host cell or can be integrated into chromosome and have a promoter at such a position that the DNA of encoding a protein having al,2-fucosyltransferase activity can be transcribed.

When a procaryote, such as a bacterium or the like, is used as the host cell, it is preferred that the used recombinant DNA can replicate autonomously in the procaryote. It is also preferred that the recombinant DNA contains a promoter, a ribosome binding sequence, the DNA encoding a protein having α1,2-fucosyltransferase activity and a transcription termination sequence. A gene regulating the promoter may also be contained in the recombinant DNA.

Examples of the expression vector include pBTrp2, pBTac1 and pBTac2 (all available from Boehringer Manheim), pKK223-3 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [*Agric. Biol. Chem*., 48, 669 (1984)], pLSA1 *[Agric. Biol. Chem.,* 53, 277 (1989)], pGEL1 [*Proc. Natl. Acad. Sci. USA,* 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS3 (FERM BP-540.8)], pGHA2 [prepared from *Escherichia coli* IGHA2 (FERM B-400), Japanese Published Unexamined Patent Application No.221091/85], pGKA2 [prepared from *Escherichia coli* IGKA2 (FERM BP-6798), pTerm2 (U.S. 4,686,191, U.S. 4,939,094, U.S. 5,160,735), pPAC31 (WO 98/12343), pSupex, pUB110, pTP5, pC194, pEG400 [*J. Bacteriol*., 172, 2392 (1990)], pGEX (manufactured by Pharmacia), pET system (manufactured by Novagen), and the like.

Any promoter can be used, so long as it can function in the host cell. Examples include promoters derived from *Escherichia coli,* phage and the like, such as trp promoter (Pₜᵣₚ), lac promoter, P_{L} promoter, P_{R} promoter, T7 promoter, and the like. Also, artificially designed and modified promoters, such as a promoter in which two Pₜᵣₚ are linked in tandem (Pₜᵣₚ×2), tac promoter, lacT7 promoter letI promoter and the like, can be used.

It is preferred to use a plasmid in which the space between Shine-Dalgarno sequence, which is the ribosome binding sequence, and the initiation codon is adjusted to an appropriate distance (for example, 6 to 18 bases).

The transcription termination sequence is not always necessary for the recombinant DNA of the present invention. However, it is preferred to provide a transcription terminating sequence just downstream of the structural gene.

Examples of the host cell include microorganisms belonging to the genera *Escherichia, Serratia, Bacillus, Brevibacterium, Corynebacterium, Microbacterium, Pseudomonas,* and the like. Specific examples include *Escherichia coli*. XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* GI698, *Escherichia coli* TB1, *Serratia ficaria, Serratia fonticola, Serratia liquefaciens, Serratia marcescens, Bacillus subtilis, Bacillus amyloliquefaciens, Corynebacterium ammoniagenes, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium glutamicum* ATCC 13869, *Corynebacterium acetoacidophilum* ATCC 13870, *Microbacterium ammoniaphilum* ATCC 15354, *Pseudomonas putida, Pseudomonas* sp. D-0110 and the like.

As the method for the introduction of the recombinant DNA, any method for introducing DNA into the above-described host cells, such as a method using a calcium ion [*Proc. Natl. Acad. Sci. USA*, 69, 2110 (1972)], a protoplast method (Japanese Published Unexamined Patent Application No. 248394/88), methods described in *Gene*, 17, 107 (1982) or *Mol. Gen. Genet*., 168, 111 (1979) and the like can be used.

When yeast is used as the host cell, examples of the expression vector include YEp13 (ATCC 37115), YEp24 (ATCC 37051), YCp50 (ATCC 37419), pHS19, pHS15 and the like.

Any promoter can be used, so long as it can function in a yeast strain. Examples include a promoter of a gene in glycolytic pathway such as hexose kinase etc., PHO5 promoter, PGK promoter, GAP promoter, ADH promoter, gal 1 promoter, gal 10 promoter, a heat shock polypeptide promoter, MFα1 promoter, CUP 1 promoter and the like.

Examples of the host cell include microorganisms belonging to the genera *Saccharomyces, Schizosaccharomyces, Kluyveromyces, Trichosporon, Schwanniomyces, Pichia, Candida* and the like. Specific examples include *Saccharomyces cerevisiae, Schizosaccharomyces pombe, Kluyveromyces lactis, Trichosporon pullulans, Schwanniomyces alluvius, Candida utilis* and the like.

As the method for the introduction of the recombinant DNA, any method for introducing DNA into yeast, such as electroporation [*Methods. Enzymol*., 194, 182 (1990)], a spheroplast method [*Proc. Natl. Acad. Sci. USA,* 75, 1929 (1978)], a lithium acetate method (*J. Bacteriol.,* 153, 163 (1983)] a method described in *Proc. Natl. Acad. Sci. USA,* 75, 1929 (1978) and the like can be used.

When an animal cell is used as the host cell, examples of the expression vector include pcDNAI and pcDM8 (manufactured by Funakoshi), pAGE107 [Japanese Published Unexamined Patent Application No. 22979/91, *Cytotechnology,* 3, 133 (1990)], pAS3-3 (Japanese Published Unexamined Patent Application No. 227075/90), pcDM8 [*Nature,* 329, 840 (1987)], pcDNAI/Amp (manufactured by Invitrogen), pREP4 (manufactured by Invitrogen), pAGE103 [*J. Biochem.,* 101, 1307 (1987)], pAGE210 and the like.

Any promoter can be used, so long as it can function in an animal cell. Examples include a promoter of IE (immediate early) gene of cytomegalovirus (CMV), an early promoter of SV40, a metallothionein promoter, a promoter of retrovirus, a heat shock promoter, SRα promoter and the like. Also, the enhancer of the IE gene of human CMV can be used together with the promoter.

Examples of the host cell include human Namalwa cell, monkey COS cell, Chinese hamster CHO cell, HBT5637 (Japanese Published Unexamined Patent Application No. 299/88) and the like.

The method for introduction of the recombinant DNA into an animal cell is not particularly limited, so long as it is a method for introducing DNA into an animal cell. Examples include electroporation *[Cytotechnology*, 3, 133 (1990)], a calcium phosphate method (Japanese Published Unexamined Patent Application No. 227075/90), lipofection [*Proc. Natl. Acad. Sci. USA*, 84, 7413 (1987)], a method described in *Virology*, 52, 456 (1973) and the like.

When an insect cell is used as the host cell, the protein can be expressed by a known method described in, for example, *Current Protocols in Molecular Biology; Baculovirus Expression Vectors, A Laboratory Manual,* W.H. Freeman and Company, New York (1992); *Bio*/*Technology*, 6, 47 (1988) or the like.

Specifically, a transfer vector and baculovirus are co-transfected into an insect cell to obtain a recombinant virus in a supernatant of the culture of its insect cell, and then an insect cell is infected with the recombinant virus to produce the protein.

Examples of the transfer vector used in the method include pVL1392, pVL1393 and pBlueBacIII (all manufactured by Invitrogen) and the like.

Examples of the baculovirus include *Autographa californica* nuclear polyhedrosis. virus which infects insects of the family *Barathra* and the like.

Examples of the insect cell include *Spodoptera frugiperda* ovary cells Sf9 and Sf21 [*Baculovirus Expression Vectors, A Laboratory Manual,* W.H. Freeman and Company, New York (1992)], *Trichoplusia ni* ovary cell High 5 (manufactured by Invitrogen) and the like.

The method for co-transfecting the transfer vector and the baculovirus for the preparation of the recombinant virus includes a calcium phosphate method (Japanese Published Unexamined Patent Application No. 227075/90), lipofection [*Proc. Natl. Acad. Sci. USA*, 84, 7413 (1987)), and the like.

When a plant cell is used as the host cell, examples of the expression vector include Ti plasmid, a tobacco mosaic virus vector and the like.

Any promoter can be used, so long as it can function in a plant cell. Examples include 35S promoter of cauliflower mosaic virus (CaMV), rice actin 1 promoter and the like.

Examples of the host cell include plant cells such as tobacco, potato, tomato, carrot, soybean, rape, alfalfa, rice, wheat, barley, *etc.,* and the like.

The method for introducing the recombinant DNA is not particularly limited, so long as it is a method for introducing DNA into a plant cell, such as the *Agrobacterium* method (Japanese Published Unexamined Patent Application No. 140885/84, Japanese Published Unexamined Patent Application No. 70080/85, WO 94/00977), electroporation (Japanese Published Unexamined Patent Application No. 251887/85), the particle gun method (Japanese Patents 2606856 and 2517813) and the like.

The protein of the present invention can be produced by culturing the thus obtained transformant which comprises a recombinant DNA comprising a DNA encoding a protein having α1,2-fucosyltransferase activity in a medium to form and accumulate the protein having α1,2-fucosyltransferase activity in the culture, and recovering the protein having α1,2-fucosyltransferase activity from the culture.

Culturing of the transformant in a medium is carried out according to the usual method as used in culturing of the host.

As a medium for culturing the transformant obtained by using, as the host, prokaryote such as *Escherichia coli* or the like or eukaryote such as yeast or the like, the medium may be either a natural medium or a synthetic medium, so long as it contains a carbon source, a nitrogen source, inorganic salts and the like which can be assimilated by the organism and the transformant can be cultured efficiently.

Any carbon source which can be assimilated by the transformant is used. Examples include carbohydrates such as glucose, fructose, sucrose, molasses containing them, starch, starch hydrolysate, *etc.*, organic acids such as acetic acid, propionic acid, *etc.*, alcohols such as ethanol, propanol, *etc.*, and the like.

Examples of the nitrogen source include ammonia, various ammonium salts of inorganic acids or organic acids such as ammonium chloride, ammonium sulfate, ammonium acetate, ammonium phosphate, *etc.*, other nitrogen-containing compounds, peptone, meat extract, yeast extract, corn steep liquor, casein hydrolysate, soybean meal and soybean meal hydrolysate, various fermented cells and hydrolysates thereof, and the like.

Examples of the inorganic salts include potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is usually carried out under aerobic conditions by shaking culture, submerged spinner culture under aeration or the like. The culturing temperature is preferably from 15 to 40°C, and the culturing time is generally from 16 hours to 7 days. The pH of the medium is preferably maintained at 3.0 to 9.0 during culturing. The pH can be adjusted using an inorganic or organic acid, an alkali solution, urea, calcium carbonate, ammonia or the like.

Also, antibiotics such as ampicillin, tetracycline, chloramphenicol and the like can be added to the medium during culturing, if necessary.

When a microorganism transformed with an expression vector containing an inducible promoter is cultured, an inducer can be added to the medium, if necessary. For example, isopropyl-β-D-thiogalactopyranoside (IPTG) or the like can be added to the medium when a microorganism transformed with an expression vector containing *lac* promoter is cultured, or indoleacrylic acid or the like can by added thereto when a microorganism transformed with an expression vector containing *trp* promoter is cultured.

Examples of the medium for culturing a transformant obtained using an animal cell as the host include generally used RPMI 1640 medium [*J.* Am. *Med. Assoc*., 199, 519 (1967)], Eagle's MEM *[Science,* 122, 501 (1952)], modified Dulbecco's MEM [*Virology*, 8, 396 (1959)], and 199 Medium [*Proc. Soc. Biol. Med.*, 73, 1 (1950)], and other media to which fetal calf serum or the like has been added to the above media and the like.

Culturing is generally carried out at pH 6 to 8 and at 30 to 40°C for 1 to 7 days in the presence of 5% CO₂.

Furthermore, if necessary, antibiotics such as kanamycin, penicillin and the like may be added to the medium during culturing.

Examples of the medium for culturing a transformant obtained using an insect cell as the host include generally used TNM-FH medium (manufactured by Pharmingen), Sf-900 II SFM (manufactured by Life Technologies), ExCell 400 and ExCell 405 (both manufactured by JRH Biosciences), Grace's Insect Medium *[Nature*, 195, 788 (1962)] and the like.

Culturing is generally carried out at pH 6 to 7 and at 25 to 30°C for 1 to 5 days.

Furthermore, if necessary, antibiotics such as gentamicin and the like may be added to the medium during culturing.

A transformant obtained using a plant cell as the host cell can be used as the cell or after differentiating to a plant cell or organ. Examples of the medium used in culturing of the transformant include Murashige and Skoog (MS) medium, White medium, media to which a plant hormone such as auxin, cytokinine or the like has been added, and the like.

Culturing is carried out generally at a pH 5 to 9 and at 20 to 40°C for 3 to 60 days.

Furthermore, if necessary, antibiotics such as kanamycin, hygromycin and the like can be added to the medium during culturing.

As described above, the protein can be produced by culturing a transformant derived from a microorganism, animal cell or plant cell containing a recombinant DNA to which a DNA encoding a protein having α1,2-fucosyltransferase activity has been inserted, according to the usual culturing method to form and accumulate the protein, and recovering the protein from the culture.

The process for producing the protein having α1,2-fucosyltransferase activity includes a method of intracellular production in a host cell, a method of extracellular secretion from a host cell, or a method of production on an outer membrane of the host cell. The method can be selected by changing the host cell employed or the structure of the protein produced.

When the protein having α1,2-fucosyltransferase activity is produced in a host cell or on an outer membrane of the host cell, the protein can be actively secreted extracellularly according to, for example, the method of Paulson *et al.* [*J. Biol. Chem*., 264, 17619 (1989)], the method of Lowe *et al.* [*Proc. Natl. Acad. Sci. USA*, 86, 8227 (1989); *Genes Develop*., 4, 1288 (1990)], or methods described in Japanese Published Unexamined Patent Application Nos. 336963/93, 823021/94, and the like.

Specifically, the protein of the present invention can be actively secreted extracellularly by expressing it in the form that a signal peptide has been added to the N-terminal side of a polypeptide containing an active site of the protein having α1,2-fucosyltransferase activity according to the recombinant DNA technique.

Furthermore, the amount produced can be increased using a gene amplification system, such as by use of a dihydrofolate reductase gene or the like according to the method described in Japanese Published Unexamined Patent Application No. 227075/90.

Moreover, the protein having α1,2-fucosyltransferase activity can be produced by redifferentiating animal or plant cells to which the gene has been introduced to prepare a gene-introduced animal individual (transgenic nonhuman animal) or plant individual (transgenic plant) and using the individuals.

When the transformant is the animal individual or plant individual, the protein can be produced by breeding or cultivating it so as to form and accumulate the protein, and recovering the protein from the animal individual or plant individual.

Examples of the process for producing the protein having α1,2-fucosyltransferase activity using the animal individual include a process for producing the protein in an animal developed by introducing a gene according to known methods [*Am. J. Clin. Nutr.*, 63, 639S (1996), *Am. J. Clin. Nutr.,* 63, 627S (1996), *Bio*/*Technology*, 9, 830 (1991)].

In the case of the animal individual, the protein can be produced by breeding a transgenic nonhuman animal to which the DNA encoding the protein having α1,2-fucosyltransferase activity has been introduced to form and accumulate the protein in the animal, and recovering the protein from the animal. Examples of the production and accumulation place in the animal include milk (Japanese Published Unexamined Patent Application No. 309192/88), egg and the like of the animal. Any promoter can be used, so long as it can function in the animal. Suitable examples include an α-casein promoter, a β-casein promoter, a β-lactoglobulin promoter, a whey acidic protein promoter, and the like, which are specific for mammary glandular cells.

Examples of the process for producing the protein of the present invention using the plant individual include a process for producing the protein having α1,2-fucosyltransferase activity by cultivating a transgenic plant, to which the DNA encoding the protein of the present invention is introduced, by a known method [*Tissue Culture (Soshiki Baiyo), 20* (1994), *Tissue Culture (Soshiki Baiyo), 21* (1994), *Trends Biotechnol*., 15, 45 (1997)] to form and accumulate the protein in the plant, and recovering the protein from the plant.

The protein having α1,2-fucosyltransferase activity produced by the transformant can be isolated and purified using the general method for isolating and purifying an enzyme. For example, when the protein of the present invention is expressed as a soluble product in the host cells, the cells are collected by centrifugation after culturing, suspended in an aqueous buffer, and disrupted using an ultrasonicator, a French press, a Manton Gaulin homogenizer, a Dynomill, or the like to obtain a cell-free extract. From the supernatant obtained by centrifuging the cell-free extract, a purified product can be obtained by the usual method used for isolating and purifying an enzyme, for example, solvent extraction, salting-out using ammonium sulfate or the like, desalting, precipitation using an organic solvent, anion exchange chromatography using a resin, such as diethylaminoethyl (DEAE)-Sepharose, DIAION HPA-75 (manufactured by Mitsubishi Chemical) or the like, cation exchange chromatography using a resin, such as S-Sepharose FF (manufactured by Pharmacia) or the like, hydrophobic chromatography using a resin, such as butyl sepharose, phenyl sepharose or the like, gel filtration using a molecular sieve, affinity chromatography, chromatofocusing, or electrophoresis, such as isoelectronic focusing or the like, alone or in combination thereof.

When the protein is expressed as an inclusion body in the host cells, the cells are collected in the same manner, disrupted and centrifuged to recover the protein as the precipitate fraction. Next, the inclusion body of the protein is solubilized with a protein-denaturing agent. The solubilized protein solution is diluted or dialyzed to lower the concentration of the protein denaturing agent in the solution. Thus, the normal tertiary structure of the protein is reconstituted. After the procedure, a purified product of the protein can be obtained by a purification/isolation method similar to the above.

When the protein having α1,2-fucosyltransferase activity or its derivatives of the protein in which a sugar chain is added to the protein are secreted out of cells, the protein or the derivatives can be collected in the culture supernatant. Specifically, the culture supernatant is obtained by treating the culture in a treatment similar to the above, such as centrifugation or the like. Then a purified product can be obtained from the supernatant using a purification/isolation method similar to the above.

Examples of the protein obtained by the above method include a protein comprising the amino acid sequence represented by SEQ ID NO:2.

Also, the protein having α1,2-fucosyltransferase activity can be produced by a chemical synthesis method, such as Fmoc (fluorenylmethyloxycarbonyl) method, tBoc (t-butyloxycarbonyl) method or the like. It can also be chemically synthesized using a peptide synthesizer manufactured by Advanced ChemTech, Perkin-Elmer, Pharmacia, Protein Technology Instrument, Synthecell-Vega, PerSeptive, Shimadzu Corporation or the like.

### [3] Preparation of fucose-containing complex carbohydrate

A fucose-containing complex carbohydrate can be produced in an aqueous medium using a culture of the transformant obtained by the culturing described in [2] or a treated product of the culture as the enzyme source.

Examples of the treated product of the culture include a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells and an enzyme preparation obtained by extracting from the cells.

The enzyme source used in the production of a fucose-containing complex carbohydrate is used at a concentration of from 0.1 mU/L to 10,000 U/L, preferably from 1 mU/L to 1,000 U/L, when the activity capable of producing 1 µmol of a fucose-containing complex carbohydrate at 37°C in one minute is defined as 1 unit (U).

Examples of the aqueous medium used in the production of a fucose-containing complex carbohydrate include water, buffers of phosphate, carbonate, acetate, borate, citrate, tris, *etc.*, alcohols such as methanol, ethanol, *etc.*, esters such as ethyl acetate, *etc.*, ketones such as acetone, *etc.*, amides such as acetamide, *etc.* and the like. Also, the microbial culture used as the enzyme source can be used as an aqueous medium.

In the production of a fucose-containing complex carbohydrate, a surfactant or an organic solvent may be added, if necessary. Any compound capable of accelerating the production of a fucose-containing complex carbohydrate may be used as the surfactant. Examples include nonionic surfactants such as polyoxyethylene octadecylamine (e.g., Nymeen S-215, manufactured by Nippon Oil & Fats), *etc.*, cationic surfactants such as cetyltrimethylammonium bromide, alkyldimethyl benzylammoniumchloride (e.g., Cation F2-40E, manufactured by Nippon Oil & Fats), *etc.,* anionic surfactants such as lauroyl sarcosinate, *etc.*, tertiary amines such as alkyldimethylamine (e.g., Tertiary Amine FB, manufactured by Nippon Oil & Fats), *etc.* and the like, which may be used alone or as a mixture of two or more. The surfactant is used generally at a concentration of from 0.1 to 50 g/L. Examples of the organic solvent include xylene, toluene, an aliphatic alcohol, acetone, ethyl acetate and the like, which are generally used at a concentration of 0.1 to 50 ml/L.

Regarding GDP-fucose as the sugar nucleotide substrate used in the production of a fucose-containing complex carbohydrate, a reaction solution obtained using activity such as a microorganism or the like or a compound purified from the reaction solution can be used, in addition to a commercial product.

The sugar nucleotide substrate is used at a concentration of 0.1 to 500 mmol/L.

As the acceptor complex carbohydrate used in the production of a fucose-containing complex carbohydrate, any substance which can become a substrate of the transferase can be used. Examples include lactose, lacto-N-neotetraose and lacto-N-tetraose, as well as an oligosaccharide consisting of 10 or less sugar having a lactose, N-acetyllactosamine, lacto-N-neotetraose, lacto-N-tetraose, Le^{x} or Le^{a} structure in the non-reducing terminal.

The acceptor complex carbohydrate is used at a concentration of 0.1 to 500 mmol/L.

In the production reaction, MgCl₂ and- the like inorganic salts, β-mercaptoethanol and the like can be added, if necessary.

The fucose-containing complex carbohydrate production reaction is carried out in an aqueous medium at pH 5 to 10, preferably pH 6 to 8, and at 20 to 50°C for 1 to 96 hours.

The amount of the fucose-containing complex carbohydrate produced in the aqueous medium can be determined in accordance with a known method *[Chemistry and Industry (Kagaku to Kogyo)*, 43, 953 (1990)].

The fucose-containing complex carbohydrate produced in the reaction solution can be recovered by the usual method using activated charcoal, an ion exchange resin or the like, and in the case of 2'-fucosyllactose, for example, it can be recovered in accordance with the method described in *J. Org. Chem*., 47, 5416 (1982).

### BRIEF DESCRIPTION OF THE DRAWINGS

Fig. 1 shows the structure of α1,2-fucosyltransferase gene expression plasmid pMG3.
Fig. 2 shows construction steps of glk, manB, manC expression plasmid pNK11.
Fig. 3 shows construction steps of gmd expression plasmid pGE19.
Fig. 4 shows construction steps of wcaG expression plasmid pGE8.

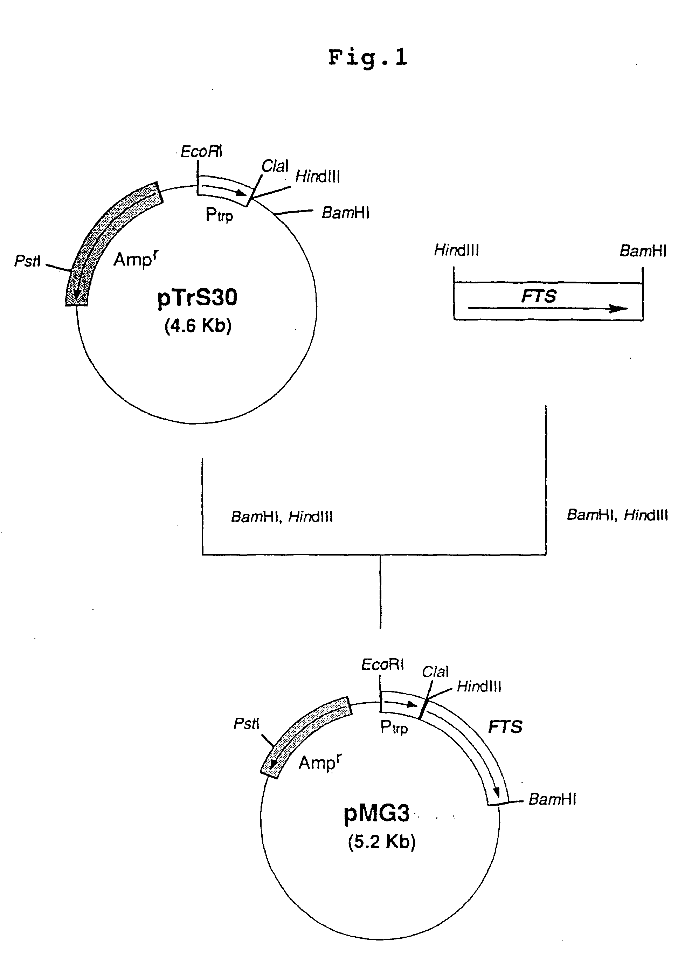

The symbols in Figs. 1 to 4 have the following meanings.
- Amp^{r} :: ampicillin resistance gene
- P_{L}:: P_{L} prompter
- P_{lac}:: lac promoter
- Pₜᵣₚ:: trp promoter
- cI857:: cI857 repressor
- glk:: glucokinase gene
- manB:: phosphomannomutase gene
- manC:: mannose-1-phosphate guanylyltransferase gene
- gmd:: GDP-marinose 4,6-dehydratase gene
- wcaG:: GKDM epimerase/reductase gene
- FTS:: α1,2-fucosyltransferase gene

### BEST MODE FOR CARRYING OUT THE INVENTION

Examples of the present invention are described below, though the present invention is not limited to these examples.

### Example 1

### Construction of a strain expressing α1,2-fucosyltransferase gene

*Bacteroides fragilis* ATCC 25285 was cultured by the known method [*Infect. Immun*., 67, 3525 (1999)]. After the culturing, chromosomal DNA of the microorganism was isolated and purified by the method described in Current *Protocols in Molecular Biology.*

The DNA represented by SEQ ID NO:3 (DNA comprising DNA represented by a nucleotide sequence of the 1st to 15th nucleotides in the nucleotide sequence represented by SEQ ID NO:1) and the DNA comprising the nucleotide sequence represented by SEQ ID NO:4 (DNA comprising DNA represented by a sequence complementary to the nucleotide sequence of the 842nd to 861st nucleotides in the nucleotide sequence represented by SEQ ID NO:1) were synthesized using Model 8905 DNA Synthesizer manufactured by Perceptive Biosystems.

PCR was carried out using the above synthetic DNA molecules as primer sets and the *Bacteroides fragilis* ATCC 25285 chromosomal DNA as the template. Using 40 µl of a reaction solution containing 0.1 µg of the chromosomal DNA, 0.5 µmol/L of each primer, 2.5 units of Pfu DNA polymerase (manufactured by Stratagene), 4 µl of 10 × buffer for Pfu DNA polymerase (manufactured by Stratagene) and 200 µmol/L of each deoxy NTP, PCR was carried out by 30 cycles, one cycle consisting of a reaction at 94°C for 1 minute, reaction at 42°C for 2 minutes and reaction at 72°C for 3 minutes.

After confirming that the desired fragment was amplified by subjecting 1/10 volume of the reaction solution to agarose gel electrophoresis, the remaining reaction solution was mixed with the same volume of TE [10 mmol/L Tris-HCl (pH 8.0), 1 mmol/L EDTA] saturated phenol/chloroform (1 vol/1 vol).

The mixed solution was centrifuged, and then the thus obtained upper layer was mixed with 2 volumes of cold ethanol and allowed to stand at -80°C for 30 minutes. The resulting solution was centrifuged to obtain a DNA precipitate.

The DNA precipitate was dissolved in 20 µl of TE. Using 5 µl of the dissolved solution, the DNA was digested with restriction enzymes *Bam*HI and *Hin*dIII and then, using a ligation kit (manufactured by Takara Shuzo), subjected to the ligation reaction at 16°C for 16 hours together with 0.2 µg of pTrS30 DNA which had been digested with the restriction enzymes *Bam*HI and *Hin*dIII.

*Escherichia coli* NM522 was transformed with the ligation reaction solution in accordance with the above known method, and the transformant was spread on LB agar medium [10 g/L bacto tryptone (manufactured by Difco), 5 g/L yeast extract (manufactured by Difco), 5 g/L NaCl (pH 7.2), 15 g/L agar] containing 50 µg/ml ampicillin, followed by culturing overnight at 30°C.

A plasmid was extracted from the thus grown transformant colony in accordance with the known method and restriction enzyme sites of the plasmid were analyzed to confirm that the desired plasmid pMG3 was constructed and *Escherichia coli* NM522/pMG3 was obtained.

### Example 2

### Construction of a strain expressing glk, manB, manC, pgm and pfkB genes:

DNAs comprising the nucleotide sequences represented by SEQ ID NOs:5 and 6 [designed based on the nucleotide sequence of glk gene described in *J. Bacteriol.,* 179, 1298 (1997)] were synthesized using Model 8905 DNA Synthesizer manufactured by Perceptive Biosystems.

PCR was carried out using the above synthetic DNA fragments as primer sets and the plasmid pNT46 (WO 98/12343) DNA as the template. Using 40 µl of a reaction solution containing 1 ng of the pNT46 DNA, 0.5 µmol/L of each primer, 2.5 units of Pfu DNA polymerase (manufactured by Stratagene), 4 µl of 10 × buffer for Pfu DNA polymerase (manufactured by Stratagene) and 200 µmol/L of each deoxy NTP, PCR was carried out by 30 cycles, one cycle consisting of a reaction at 94°C for 1 minute, reaction at 42°C for 2 minutes and reaction at 72°C for 3 minutes.

After confirming that the desired fragment was amplified by subjecting 1/10 volume of the reaction solution to agarose gel electrophoresis, the remaining reaction solution was mixed with the same volume of TE saturated phenol/chloroform.

The mixed solution was centrifuged, and then the thus obtained upper layer was mixed with 2 volumes of cold ethanol and allowed to stand at -80°C for 30 minutes. The resulting solution was centrifuged to obtain a DNA precipitate.

The DNA precipitate was dissolved in 20 µl of TE. Using 5 µl of the dissolved solution, the DNA was digested with restriction enzymes *Bgl*II and *Sal*I and the DNA fragments were separated by agarose gel electrophoresis, and then a DNA fragment of 1.3 kb containing glk gene was recovered by using Gene Clean II Kit (purchased from Funakoshi).

After 0.2 µg of plasmid pNK7 expressing manB and manC (WO 98/12343) was digested with restriction enzymes *Bam*HI and *Sal*I, the DNA fragments were separated by agarose gel electrophoresis and then a -fragment of 8.2 kb was recovered in the same manner.

The fragments of 1.3 kb and 8.2 kb were subjected to the ligation reaction at 16°C for 16 hours using a ligation kit. *Escherichia coli* NM522 was transformed with the ligation reaction solution in accordance with the above known method, and the transformant was spread on LB agar medium containing 50 µg/ml ampicillin, followed by culturing overnight at 30°C.

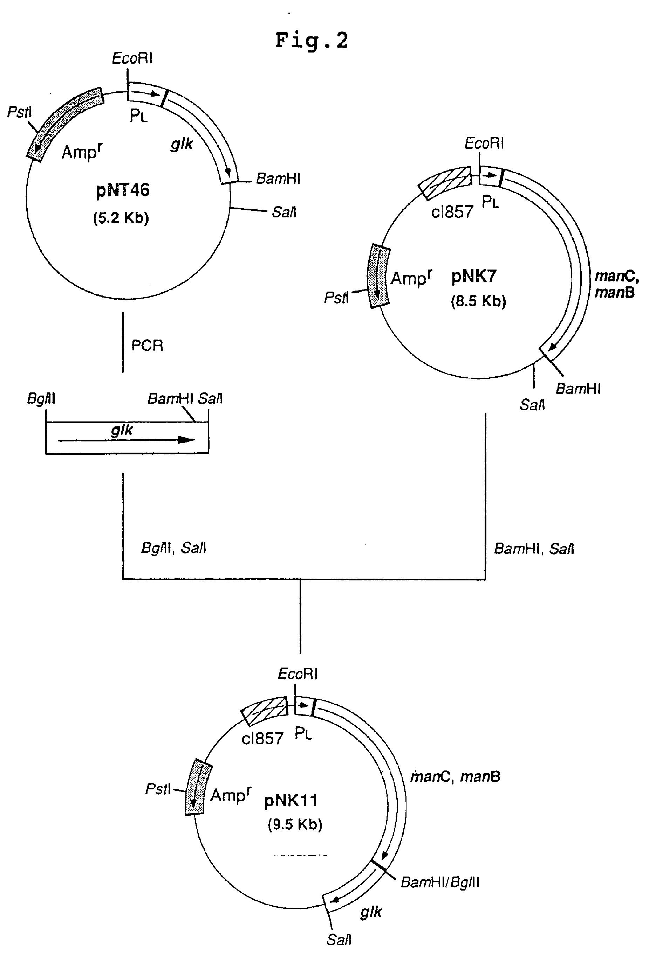

By extracting plasmids from the thus grown transformant colonies in accordance with the above known method, plasmid pNK11 expressing glk, manB and manC genes was obtained. The structure of the plasmid was confirmed by restriction enzyme digestion (Fig. 2).

*Escherichia coli* NM522/pNT55 (WO 98/12343) was transformed with the thus obtained pNK11 DNA in accordance with the known method, and the transformant was spread on LB agar medium containing 50 µg/ml ampicillin and 10 µg/ml chloramphenicol, followed by culturing overnight at 30°C. By selecting the thus grown transformants, *Escherichia coli* NM522/pNK11/pNT55 was obtained as a strain simultaneously expressing glk, manB, manC, pgm and pfkB genes.

### Example 3

### Construction of a strain in which a gmd gene derived from Escherichia coli is expressed:

After culturing *Escherichia coli* W3110 (ATCC 27325) by the method described in *Current Protocols in Molecular Biology,* chromosomal DNA of the microorganism was isolated and purified.

The PCR was carried out in accordance with the method described in Example 1, using DNA comprising the nucleotide sequences represented by SEQ ID NOs:7 and 8 [designed based on the nucleotide sequence of gmd gene described in *J. Bacteriol*., 178, 4885 (1996)] synthesized using Model 8905 DNA Synthesizer manufactured by Perceptive Biosystems as primer sets, and 0.1 µg of the chromosomal DNA of *Escherichia coli* W3110 (ATCC 27325) as the template.

After confirming that the desired fragment was amplified by subjecting 1/10 volume of the reaction solution to agarose gel electrophoresis, the remaining reaction solution was mixed with the same volume of TE saturated phenol/chloroform.

The mixed solution was centrifuged, and then the thus obtained upper layer was mixed with 2 volumes of cold ethanol and allowed to stand at -80°C for 30 minutes and then centrifuged to obtain a DNA precipitate.

The DNA precipitate was dissolved in 20 µl of TE. Using 5 µl of the dissolved solution, the DNA was digested with restriction enzymes *Hin*dIII and *Xba*I, DNA fragments were separated by agarose gel electrophoresis, and then a DNA fragment of 1.1 kb containing gmd gene was recovered using Gene Clean II Kit.

PCR was carried out in accordance with the method described in Example 2, using DNA fragments comprising the nucleotide sequences represented by SEQ ID NOs:9 and 10 (designed based on the nucleotide sequence described in Japanese Published Unexamined Patent Application No. 110600/83) synthesized using Model 8905 DNA Synthesizer manufactured by Perceptive Biosystems as primer sets, and a *Trp* promoter-containing plasmid pTrS30 as the template.

After confirming that the desired fragment was amplified by subjecting 1/10 volume of the reaction solution to agarose gel electrophoresis, the remaining reaction solution was mixed with the same volume of TE saturated phenol/chloroform.

The mixed solution was centrifuged, and then the thus obtained upper layer was mixed with 2 volumes of cold ethanol and allowed to stand at -80°C for 30 minutes and then centrifuged to obtain a DNA precipitate.

The DNA precipitate was dissolved in 20 µl of TE. Using 5 µl of the dissolved solution, the DNA was digested with restriction enzymes *Eco*RI and *Xba*I, the DNA fragments were separated by agarose gel electrophoresis, and then a DNA fragment of 0.4 kb was recovered in the same manner.

After 0.2 µg of pBluescript II SK+ was digested with restriction enzymes *Eco*RI and *Hind*III, the DNA fragments were separated by agarose gel electrophoresis, and then a DNA fragment of 3.0 kb was recovered in the same manner.

The fragments of 1.1 kb, 0.4 kb and 3.0 kb were subjected to ligation reaction at 16°C for 16 hours using a ligation kit.

Escherichia *coli* NM522 was transformed with the ligation reaction solution in accordance with the above known method, and the transformant was spread on LB agar medium containing 50 µg/ml ampicillin, followed by culturing overnight at 30°C.

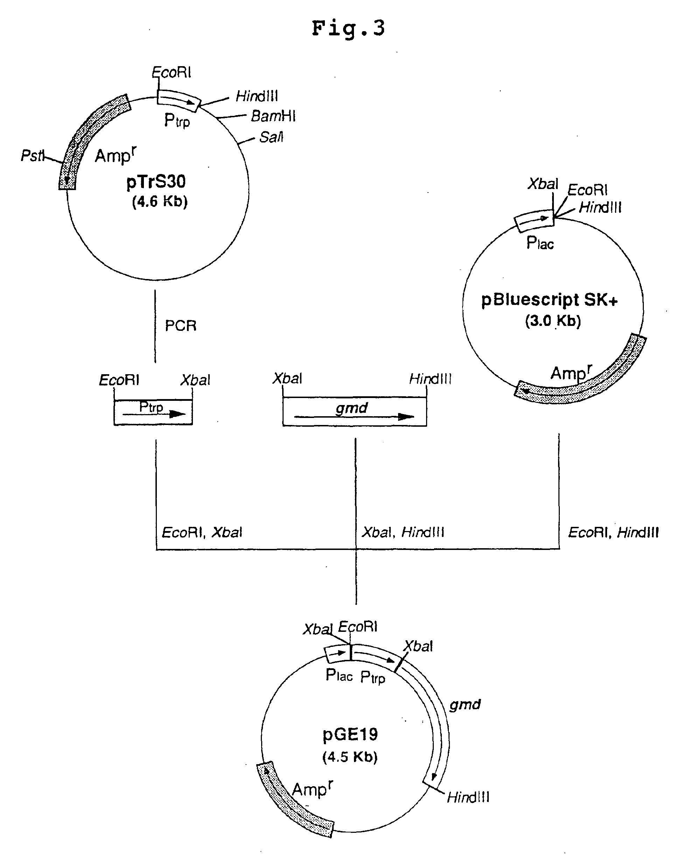

Plasmids were extracted from the thus grown transformant colonies in accordance with the above known method to obtain expression plasmid pGE19. The structure of the plasmid was confirmed by restriction enzyme digestion (Fig. 3).

### Example 4

### Construction of a strain in which a wcaG gene derived from Escherichia coli is expressed:

The PCR was carried out in accordance with the method described in Example 1, using DNAs comprising the nucleotide sequences represented by in SEQ ID NOs:11 and 12 [designed based on the nucleotide sequence of wcaG gene described in *J. Bacteriol*., 178, 4885 (1996)] synthesized using Model 8905 DNA Synthesizer manufactured by Perceptive Biosystems as the primer sets, and chromosomal DNA of *Escherichia coli* W3110 (ATCC 27325) as the template.

After confirming that the desired fragment was amplified by subjecting 1/10 volume of the reaction solution to agarose gel electrophoresis, the remaining reaction solution was mixed with the same volume of TE saturated phenol/chloroform.

The mixed solution was centrifuged, and then the thus obtained upper layer was mixed with 2 volumes of cold ethanol and allowed to stand at -80°C for 30 minutes, and then centrifuged to obtain a DNA precipitate.

The DNA precipitate was dissolved in 20 µl of TE. Using 5 µl of the dissolved solution, the DNA was digested with restriction enzymes *Cla*I and *Xho*I, the DNA fragments were separated by agarose gel electrophoresis, and then a DNA fragment of 1.0 kb containing wcaG gene was recovered using Gene Clean II Kit.

After 0.2 µg of pPAC31 was digested with restriction enzymes *Cla*I and *Sal*I, the DNA fragments were separated by agarose gel electrophoresis, and then a DNA fragment of 5.2 kb was recovered in the same manner.

The fragments of 1.0 kb and 5.2 kb was subjected to ligation reaction at 16°C for 16 hours using a ligation kit.

*Escherichia coli* NM522 was transformed with the ligation reaction solution in accordance with the above known method, and the transformant was spread on LB agar medium containing 50 µg/ml ampicillin, followed by culturing overnight at 30°C.

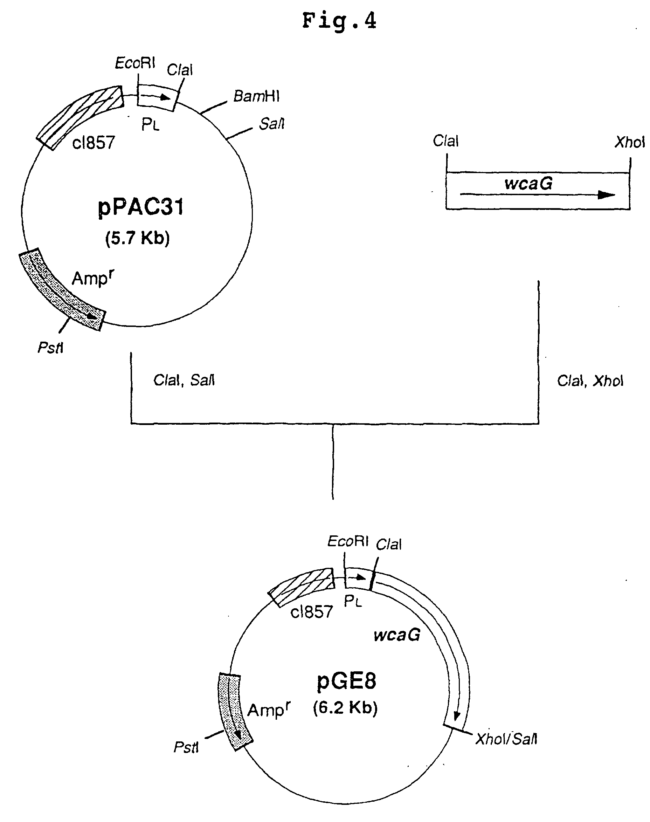

Plasmids were extracted from the thus grown transformant colonies in accordance with the above known method to obtain expression plasmid pGE8. The structure of the plasmid was confirmed by restriction enzyme digestion (Fig. 4).

### Example 5

### Production of 2'-fucosyllactose (1):

*Escherichia coli* NM522/pMG3 obtained in Example 1 was inoculated into 8 ml of LB medium containing 50 µg/ml ampicillin in a large test tube and cultured at 28°C for 17 hours. The resulting culture was inoculated into 8 ml of LB medium containing 50 µg/ml ampicillin in a large test tube in an amount of 1% and cultured at 37°C for 5 hours. Wet cells were obtained by centrifuging 0.1 ml of the culture. The wet cells could be stored at -20°C, if necessary, and it was able to use them by thawing before to use.

A reaction solution of 0.1 ml containing the wet cells (corresponding to 0.1 ml of culture), 50 mM citrate buffer (pH 7.0), 10 mmol/L MnCl₂, 10 mmol/L lactose, 10 mmol/L GDP-fucose and 0.4% Nymeen S-215 was prepared and the reaction was carried out at 37°C for 16 hours.

After completion of the reaction, the reaction product was analyzed using a carbohydrate analysis system (DX-500) manufactured by Dionex under the following analyzing conditions to confirm that 0.13 mmol/L (64 mg/L) 2'-fucosyllactose was formed and accumulated in the reaction solution.

### Analyzing conditions:

Column: CarboPAC PA10
Eluent: A; H₂O, B; 500 mmol/L NaOH
Gradient: 8 to 20% B in 21 min
Detector: Pulsed amperometry detector

### Example 6

### Production of 2'-fucosyllactose (2):

*Escherichia coli* NM522/pMG3 obtained in Example 1 was inoculated into 125 ml of LB medium [10 g/L Bacto-tryptone (manufactured by Difco), 5 g/L yeast extract (manufactured by Difco), 5 g/L NaCl (pH 7.3)] containing 50 µg/ml ampicillin in a 1-L Erlenmeyer flask with baffles and cultured at 28°C and at 220 rpm for 17 hours. The resulting culture (125 ml) was inoculated into 2.5 L of TB medium [10 g/L glucose, 12 g/L Bacto-tryptone (manufactured by Difco), 24 g/L yeast extract (manufactured by Difco), 2.3 g/L KH₂PO₄ and 12.5 g/L K₂HPO₄ (pH not adjusted)] containing 50 mg/L ampicillin in a 5-L jar fermenter and cultured at 37°C for 6 hours and at 600 rpm and 2.5 L/min aeration. During the culturing, the medium pH was controlled at 7.0 using 28% aqueous ammonia, and glucose was added in case of necessity. The resulting culture was centrifuged to obtain wet cells. The wet cells could be stored at -20°C, if necessary, and it was able to use them by thawing before to use.

*Escherichia coli* NM522/pNK11/pNT55 obtained in Example 2 was inoculated into 125 ml of LB medium containing 50 µg/ml ampicillin and 10 µg/ml chloramphenicol in a 1-L Erlenmeyer flask with baffles and cultured at 28°C and at 220 rpm for 17 hours. The resulting culture (125 ml) was inoculated into 2.5 L of TB medium containing 50 mg/L ampicillin and 10 mg/L chloramphenicol in a 5-L jar fermenter and cultured at 30°C for 4 hours with stirring at 600 rpm and 2.5 L/min aeration, followed by culturing at 40°C for 3 hours. During the culturing, the medium pH was controlled at 7.0 using 28% aqueous ammonia, and glucose was added in case of necessity. The resulting culture was centrifuged to obtain wet cells. The wet cells could be stored at -20°C, if necessary, and it was able to use them by thawing before to use.

*Escherichia coli* NM522/pGE19 obtained in Example 3 was inoculated into 125 ml of LB medium containing 50 µg/ml ampicillin in a 1-L Erlenmeyer flask with baffles and cultured at 28°C with stirring at 220 rpm for 17 hours. The resulting culture (125 ml) was inoculated into 2.5 L of TB medium containing 50 µg/ml ampicillin in a 5-L jar fermenter and cultured at 37°C for 6 hours with stirring at 600 rpm and 2.5 L/min aeration. During the culturing, the medium pH was controlled at 7.0 using 28% aqueous ammonia, and glucose was added in case of necessity. The resulting culture was centrifuged to obtain wet cells. The wet cells could be stored at -20°C, if necessary, and it was able to use them by thawing prior to use.

*Escherichia coli* NM522/pGE8 obtained in Example 4 was inoculated into 125 ml of LB medium containing 50 µg/ml ampicillin in a 1-L Erlenmeyer flask with baffles and cultured at 28°C with stirring at 220 rpm for 17 hours. The resulting culture (125 ml) was inoculated into 2.5 L of TB medium containing 50 µg/ml ampicillin in a 5-L jar fermenter and cultured at 30°C for 4 hours with stirring at 600 rpm and 2.5 L/min aeration, followed by culturing at 40°C for 3 hours. During the culturing, the medium pH was controlled at 7.0 using 28% aqueous ammonia, and glucose was added in case of necessity. The resulting culture was centrifuged to obtain wet cells. The wet cells could be stored at -20°C, if necessary, and it was able to use them by thawing before to use.

*Corynebacterium ammoniagenes* ATCC 21170 was inoculated into 25 ml of a liquid medium consisting of 50 g/L glucose, 10 g/L polypeptone (manufactured by Nihon Pharmaceutical), 10 g/L yeast extract (manufactured by Oriental Yeast), 5 g/L urea, 5 g/L (NH₄)₂SO₄, 1 g/L KH₂PO₄, 3 g/L K₂HPO₄, 1 g/L MgSO₄·7H₂O, 0.1 g/L CaCl₂·2H₂O, 10 mg/L FeSO₄·7H₂O, 10 mg/L ZnSO₄·7H₂O, 20 mg/L MnSO₄·4-6H₂O, 20 mg/L L-cysteine, 10 mg/L calcium D-pantothenate, 5 mg/L vitamin B₁, 5 mg/L nicotinic acid and 30 µg/L biotin (adjusted to pH 7.2 with 10 mol/L NaOH) in a 300-ml Erlenmeyer flask with baffles and cultured at 28°C with stirring at 220 rpm for 24 hours.

The resulting culture (20 ml) was inoculated into 250 ml of a liquid medium having the same composition of the above in a 2-L Erlenmeyer flask with baffles and cultured at 28°C with stirring at 220 rpm for 24 hours. The thus obtained culture was used as a seed culture.

The resulting seed culture (250 ml) was inoculated into 2.25 L of a liquid medium consisting of 150 g/L glucose, 5 g/L meat extract (manufactured by Kyokuto Seiyaku), 10 g/L KH₂PO₄, 10 g/L K₂HPO₄, 10 g/L MgSO₄ · 7H₂O, 0.1 g/L CaCl₂·2H₂O, 20 mg/L FeSO₄·7H₂O, 10 mg/L ZnSO₄·7H₂O, 20 mg/L MnSO₄·4-6H₂O (separate sterilization), 15 mg/L β-alanine (separate sterilization), 20 mg/L L-cysteine, 100 µg/L biotin, 2 g/L urea and 5 mg/L vitamin B₁ (separate sterilization) (adjusted to pH 7.2 with 10 mol/L NaOH) in a 5-L jar fermenter and cultured at 32°C for 24 hours with stirring at 600 rpm and 2.5 L/min aeration. During the culturing, the medium pH was controlled at 6.8 using 28% aqueous ammonia.

The resulting culture was centrifuged to obtain wet cells. The wet cells can be stored at -20°C, if necessary, and it was able to use them by thawing before to use.

A reaction solution (30 ml) consisting of 25 g/L *Escherichia coli* NM522/pNK11/pNT55 wet cells, 15 g/L *Escherichia coli* NM522/PGE19 wet cells, 15 g/L *Escherichia coli* NM522/pGE8 wet cells, 50 g/L *Escherichia coli* NM522/pGT35 wet cells, 150 g/L *Corynebacterium ammoniagenes* ATCC 21170 wet cells, 100 g/L fructose, 30 g/L mannose, 100 g/L lactose, 30 g/L GMP, 25 g/L KH₂PO₄, 5 g/L MgSO₄·7H₂O, 5 g/L phytic acid, 5 g/L Nymeen S-215 and 10 ml/L xylene was put into a 200-ml beaker, and the reaction solution was stirred using a magnetic stirrer (900 rpm) for carrying out the reaction at 32°C for 48 hours. During the reaction, the pH of the reaction solution was kept to 7.2 using 4 mol/L NaOH, and fructose and KH₂PO₄ were added in case of necessity.

After completion of the reaction, the reaction product was analyzed using a carbohydrate analysis system (DX-500) manufactured by Dionex (DX-500) (the analyzing conditions are the same as described in Example 5) to confirm that 3.2 g/L 2'-fucosyllactose was formed and accumulated in the reaction solution.

### Example 7

### Production of fucosyllacto-N-neotetraose:

*Escherichia coli* NM522/pMG3 obtained in Example 1 was inoculated 8 ml of LB medium containing 50 µg/ml ampicillin in a large test tube and cultured at 28°C for 17 hours.

The resulting culture was inoculated into 8 ml of LB medium containing 50 µg/ml ampicillin in a large test tube in an amount of 1% and cultured at 37°C for 5 hours.

The resulting culture (0.1 ml) was centrifuged to obtain wet cells. The wet cells could be stored at -20°C, if necessary, and it was able to use them by thawing before to use.

A reaction solution (0.1 ml) consisting of the wet cells (corresponding to 0.1 ml culture), 50 mmol/L citrate buffer (pH 7.0), 10 mmol/L MnCl₂, 10 mmol/L GDP-fucose, 0.4% Nymeen S-215 and 10 mmol/L lacto-N-neotetraose was prepared and the reaction was carried out at 37°C for 16 hours.

After completion of the reaction, the reaction product was analyzed using a carbohydrate analysis system (DX-500) manufactured by Dionex (the analyzing conditions are as described in Example 5) to confirm that 4.6 nmol/L (4 mg/L) fucosyllacto-N-neotetraose was formed and accumulated in the reaction solution.

### INDUSTRIAL APPLICABILITY

According to the present invention, α1,2-fucosyltransferase can be produced in a large amount, and fucose-containing complex carbohydrates such as 2'-fucosyllactose and the like can be produced efficiently using the enzyme.

### FREE TEXT OF SEQUENCE LISTING

SEQ ID NO:3 - Explanation of synthetic sequence: Synthetic DNA
SEQ ID NO:4 - Explanation of synthetic sequence: Synthetic DNA
SEQ ID NO:5 - Explanation of synthetic sequence: Synthetic DNA
SEQ ID NO:6 - Explanation of synthetic sequence: Synthetic DNA
SEQ ID NO:7 - Explanation of synthetic sequence: Synthetic DNA
SEQ ID NO:8 - Explanation of synthetic sequence: Synthetic DNA
SEQ ID NO:9- Explanation of synthetic sequence: Synthetic DNA
SEQ ID NO:10 - Explanation of synthetic sequence: Synthetic DNA
SEQ ID NO:11 - Explanation of synthetic sequence: Synthetic DNA
SEQ ID NO:12 - Explanation of synthetic sequence: Synthetic DNA

## Claims

1. A process for producing a fucose-containing complex carbohydrate, which comprises selecting, as an enzyme source, a culture of a transformant expressing a protein having α1,2-fucosyltransferase activity derived from a microorganism belonging to the genus *Bacteroides* or a treated product of the culture; allowing the enzyme source, an acceptor complex carbohydrate and guanosine diphosphofucose (hereinafter referred to as "GDP-fucose") to be present in an aqueous medium; transferring fucose to the acceptor complex carbohydrate by α1,2 linkage to form and accumulate the fucose-containing complex carbohydrate in the aqueous medium; and recovering the fucose-containing complex carbohydrate from the aqueous medium.

2. A process for producing the fucose-containing complex carbohydrate according to claim 1, which comprises selecting, as enzyme sources, a culture of a microorganism capable of producing guanosine 5'-triphosphate (hereinafter referred to as "GTP") from a precursor or a treated product of the culture, a culture of a microorganism capable of producing GDP-fucose from a sugar and GTP or a treated product of the culture and a culture of a transformant expressing a protein having α1,2-fucosyltransferase activity derived from a microorganism belonging to the genus *Bacteroides* or a treated product of the culture; allowing the enzyme sources, the precursor, the sugar and an acceptor complex carbohydrate to be present in an aqueous medium; allowing the fucose-containing complex carbohydrate to form and accumulate in the aqueous medium; and recovering the fucose-containing complex carbohydrate from the aqueous medium.

3. A process for producing a protein having α1,2-fucosyltransferase activity, which comprises culturing a transformant expressing a protein having α1,2-fucosyltransferase activity derived from a microorganism belonging to the genus *Bacteroides* in a medium; allowing the protein having α1,2-fucosyltransferase activity to form and accumulate in the culture; and recovering the protein from the culture.

4. The process according to any one of claims 1 to 3, wherein the transformant is a transformant obtained by introducing a recombinant DNA into a microorganism.

5. The process according to claim 4, wherein the microorganism is a microorganism belonging to the genus *Escherichia.*

6. The process according to claim 5, wherein the microorganism belonging to the genus *Escherichia* is *Escherichia coli.*

7. The process according to claim 4, wherein the recombinant DNA comprises a DNA encoding a protein having α1,2-fucosyltransferase activity derived from a microorganism belonging to the genus *Bacteroides.*

8. The process according to claim 7, wherein the DNA encoding a protein having α1,2-fucosyltransferase activity derived from a microorganism belonging to the genus *Bacteroides* is a DNA described in the following (1) or (2):
(1) a DNA comprising the nucleotide sequence represented by SEQ ID NO:1;
(2) a DNA which comprises a nucleotide sequence in which at least one nucleotide in the DNA consisting of the nucleotide sequence represented by SEQ ID NO:1 is deleted, substituted or added, and encodes a protein having α1,2-fucosyltransferase activity.

9. The process for producing a fucose-containing complex carbohydrate according to claim 1 or 2, wherein the acceptor complex carbohydrate is a complex carbohydrate containing an oligosaccharide consisting of 10 or less sugar having galactose in the non-reducing terminal.

10. The process for producing a fucose-containing complex carbohydrate according to claim 9, wherein the oligosaccharide has a lactose, N-acetyllactosamine, lacto-N-neotetraose, lacto-N-tetraose, Le^{x} or Le^{a} structure in the non-reducing terminal.

11. The process for producing a fucose-containing complex carbohydrate according to claim 1 or 2, wherein the treated product of the culture is a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically ground product of the cells, a solvent-treated product of the cells, an enzyme-treated product of the cells, a protein fraction of the cells, an immobilized product of the cells or an enzyme preparation obtained by extracting the cells.

12. The process for producing a fucose-containing complex carbohydrate according to claim 2, wherein the precursor is guanine, xanthine, hypoxanthine, guanosine, xanthosine, inosine, guanosine-5'-monophosphate, xanthosine-5'-monophosphate or inosine-5'-monophosphate.

13. The process for producing a fucose-containing complex carbohydrate according to claim 2, wherein the sugar is selected from glucose, fructose and mannose.

14. The process for producing a fucose-containing complex carbohydrate according to claim 2, wherein the microorganism capable of producing GTP from a precursor is a microorganism selected from microorganisms belonging to the genus *Corynebacterium.*

15. The process for producing a fucose-containing complex carbohydrate according to claim 14, wherein the microorganism belonging to the genus *Corynebacterium* is *Corynebacterium ammoniagenes.*

16. The process for producing a fucose-containing complex carbohydrate according to claim 2, wherein the microorganism capable of producing GDP-fucose from a sugar and GTP consists of at least one microorganism.

17. The process for producing a fucose-containing complex carbohydrate according to claim 16, wherein the microorganism is at least one microorganism selected from microorganisms belonging to the genus *Escherichia* and genus *Corynebacterium.*

18. The process for producing a fucose-containing complex carbohydrate according to claim 17, wherein the microorganism belonging to the genus *Escherichia* is *Escherichia coli.*

19. The process for producing a fucose-containing complex carbohydrate according to claim 17, wherein the microorganism belonging to the genus *Corynebacterium* is *Corynebacterium ammoniagenes*.

20. The process for producing a fucose-containing complex carbohydrate according to claim 2, wherein the microorganism capable of producing GDP-fucose from a sugar and GTP is a microorganism having potent activity of at least one enzyme selected from the group consisting of glucokinase, phosphomannomutase, mannose-1-phosphate guanylyltransferase, phosphoglucomutase, phosphofructokinase, GDP-mannose 4,6-dehydratase and GKDM epimerase/reductase.

21. The process for producing a fucose-containing complex carbohydrate according to claim 20, wherein the microorganism is at least one microorganism containing a recombinant DNA which comprises a DNA fragment comprising a gene selected from the group consisting of a gene encoding glucokinase (hereinafter referred to as "glk gene"), a gene encoding phosphomannomutase (hereinafter referred to as "manB gene"), a gene encoding mannose-1-phosphate guanylyltransferase (hereinafter referred to as "manC gene"), a gene encoding phosphoglucomutase (hereinafter referred to as "pgm gene"), a gene encoding phosphofructokinase (hereinafter referred to as "pfk gene"), a gene encoding GDP-mannose 4,6-dehydratase (hereinafter referred to as "gmd gene") and a gene encoding GKDM epimerase/reductase (hereinafter referred to as "wcaG gene"); and a vector.

22. The process for producing a fucose-containing complex carbohydrate according to claim 21, wherein the glk gene, the manB gene, the manC gene, the pgm gene, the pfk gene, the gmd gene and the wcaG gene are genes derived from *Escherichia coli*.

23. An agent for synthesizing a fucose-containing complex carbohydrate, which comprises a protein selected from the following (1) to (3) as an active ingredient:
(1) a protein having α1,2-fucosyltransferase activity derived from a microorganism belonging to the genus *Bacteroides*;
(2) a protein comprising the amino acid sequence represented by SEQ ID NO:2;
(3) a protein which comprises an amino acid sequence in which at least one amino acid of the protein consisting of the amino acid sequence represented by SEQ ID NO:2 is deleted, substituted or added, and has α1,2-fucosyltransferase activity.
